# FASCICULE DE BREVET EUROPEEN

(11) **EP 1 469 796 B1**
(45) Date de publication et mention de la délivrance du brevet: **16.05.2007**
(21) Numéro de dépôt: 03720606.7
(22) Date de dépôt: 03.02.2003
(51) Int. Cl.: A61F 2/16

(54) **IMPLANT INTRACAPSULAIRE ACCOMODATIF**
INTRAKAPSULARES, AKKOMODATIVES IMPLANTAT
ACCOMMODATIVE INTRACAPSULAR IMPLANT

(30) Priorité: 01.02.2002 FR 0201240
(43) Date de publication de la demande: 27.10.2004
(73) Titulaire: HumanOptics AG, 91054 Erlangen (DE)
(72) Inventeur: HANNA, Khalil, F-75007 Paris (FR)
(74) Mandataire: Rau, Albrecht
(86) Numéro de dépôt international: PCT/FR2003/000320
(87) Numéro de publication internationale: WO 2003/063738

(56) Documents cités:
- WO-A-91/06259
- US-A- 4 556 998
- US-A- 4 710 194
- US-A- 4 888 012
- US-A- 5 066 301
- US-A- 5 135 592

## Description

Le remplacement du cristallin naturel par un cristallin artificiel (implant) est aujourd'hui couramment pratiqué, notamment sur des sujets atteints de cataracte.

L'acte chirurgical le plus pratiqué consiste à retirer la matière du cristallin naturel contenue dans le sac capsulaire en préservant l'intégrité de ce dernier (à l'exception de la partie centrale de sa paroi antérieure) qui demeure alors le réceptacle de l'implant placé en remplacement de la matière naturelle retirée.

### ARRIERE PLAN DE L'INVENTION

Il existe aujourd'hui de nombreux implants dont la plupart permet au sujet de recouvrer une vision de loin satisfaisante sans possibilité d'accommoder pour une vision de près.

L'accommodation naturelle fait l'objet de nombreuses études afin d'identifier les phénomènes qui sont mis en oeuvre pour tenter de les transposer aux implants. Le rôle du sac capsulaire dans l'accomodation est extrêmement important, notamment comme élément de transmission à la matière cristalline des forces engendrées par le muscle ciliaire dans l'un ou l'autre de ses états relâché ou contracté, auquel le sac capsulaire est relié par les fibres zonulaires.

Les plus récents travaux à ce jour d'une part sur l'accommodation et d'autre part sur les implants accommodatifs placés dans le sac capsulaire ont montré que le sac capsulaire et la matière cristalline disposent d'une élasticité propre qui imprime au cristallin naturel une forme modifiable selon les équilibres de forces obtenus entre l'état de tension des fibres zonulaires, l'élasticité du sac capsulaire et l'élasticité de la matière cristalline. La perte du pouvoir accommodatif semble résulter d'une modification du module d'élasticité de (au vu des forces mises en jeu) voisin de sa forme pour la vision de loin, d'où le phénomène de presbytie.

Le WO 01/19288 A1 révèle un cristallin intraoculaire avec une zone marginale autour de celui-ci.

L'un des buts de l'invention est de proposer un implant intracapsulaire accommodatif qui reproduise les mécanismes mis en jeu lors de l'accommodation.

A cet effet l'invention a donc pour objet un implant intracapsulaire accommodatif comprenant une partie optique centrale et au moins deux bras haptiques dont les extrémités libres sont conformées pour coopérer avec des portions de zone équatoriale du sac capsulaire, caractérisé en ce que la partie optique est formée par la réunion de deux corps élastiquement déformables l'un en forme d'enveloppe et l'autre en forme de noyau, l'enveloppe présentant une surface extérieure avec une face antérieure convexe qui possède, lorsque l'enveloppe est vide un premier rayon de courbure et lorsque l'enveloppe emprisonne le noyau, un deuxième rayon de courbure différent du premier rayon de courbure.

Il est ainsi reproduit, dans la partie optique de l'implant une structure élastique dans laquelle règnent deux champs de forces antagonistes qui donnent à la structure une forme déterminée à l'équilibre. Une perturbation de cet équilibre par l'addition de forces extérieures se traduit par une modification de la forme de la partie optique, notamment de la courbure de sa face antérieure et donc de sa puissance optique. On dispose alors d'un implant accommodatif essentiellement par modification de la forme de la partie optique (avec également un déplacement de celle-ci le long de l'axe optique de l'oeil) contrairement à tous les implants connus qui n'accommodent que par déplacement.

D'autres caractéristiques de l'invention apparaîtront au cours de la description donnée ci-après à titre d'exemple d'un mode de réalisation.

Il sera fait référence aux dessins annexés parmi du sac capsulaire. On dispose alors d'un implant accommodatif essentiellement par modification de la forme de la partie optique (avec également un déplacement de celle-ci le long de l'axe optique de l'oeil) contrairement à tous les implants connus qui n'accommodent que par déplacement d'un lentille optique.

D'autres caractéristiques de l'invention apparaîtront au cours de la description donnée ci-après à titre d'exemple d'un mode de réalisation. -

### BREVE DESCRIPTION DES DESSINS

Il sera fait référence aux dessins annexés parmi lesquels :
- la figure 1 est une coupe diamétrale de l'élément enveloppe de l'implant selon l'invention,
- la figure 2 est une coupe diamétrale de l'élément noyau de l'implant selon l'invention,
- la figure 3 est une coupe diamétrale de l'implant selon l'invention, l'enveloppe emprisonnant le noyau,
- la figure 4 est une coupe diamétrale de l'implant dans sa configuration correspondant à un état accommodé,
- les figures 5 et 6 illustrent une variante de réalisation des figures 1 et 2,
- la figure 7 est une vue partielle de dessus de l'implant selon l'invention.

### DESCRIPTION DETAILLEE DE L'INVENTION

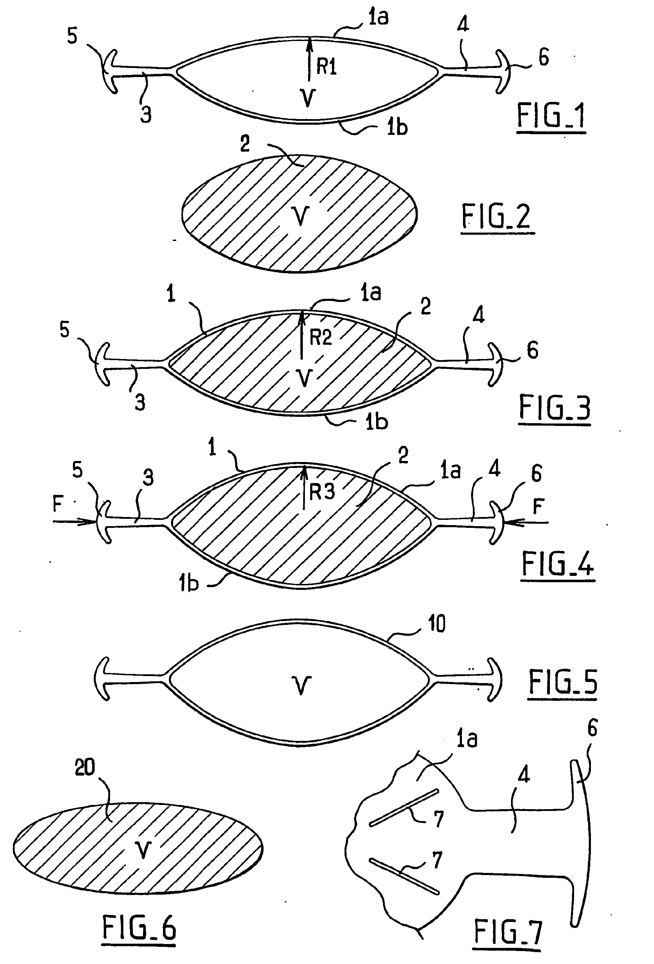

L'implant comprend deux parties : une enveloppe 1 représentée vide à la figure 1 et un noyau 2 représenté à son état libre à la figue 2. L'enveloppe 1 forme une coque en matière élastique pourvue de deux bras radiaux 3 et 4 pourvus de semelles 5 et 6 en extrémité. Il s'agit de la partie haptique de l'implant qui prend appui sur des zones équatoriales du sac capsulaire de l'oeil qui n'est pas représenté.

Le volume intérieur V de la coque 1 est identique au volume V du noyau 2. Ce noyau 2 est réalisé dans un matériau élastomère, donc élastiquement déformable de sorte que lorsqu'il est introduit dans la coque 2 celle-ci est gonflée par le noyau et le noyau est aplati par la coque, car la forme à l'état libre du noyau 2 est plus renflée que la forme du volume V de la coque. La forme finale atteinte par la partie optique de l'implant (que constitue le volume V de la coque rempli du noyau 2) est représentée à la figure 3. Le volume intérieur de la coque peut être légèrement supérieur à celui du noyau en matériau élastomère dans la mesure où cet excès de volume n'a qu'une influence négligeable sur l'interaction de l'enveloppe et du noyau lorsqu'ils sont assemblés. La mise en place du noyau dans la coque est réalisée par tout moyen : injection au travers d'une fente équatoriale de la coque, réunion de deux demi-coques autour du noyau...

Dans cette forme, le rayon de courbure R2 de la face extérieure antérieure 1a de la coque 1 est différent de ce même rayon R1 lorsque la coque est vide. Ici le rayon R2 est plus petit que le rayon R1. La forme atteinte à la figure 3 est le résultat de l'équilibre de deux champs de forces antagonistes nés de la compression élastique du noyau 2 par la coque 1 et de l'expansion élastique de la coque 1 par le noyau 2.

En provoquant une altération de cet équilibre par un champ de forces extérieur F (voir figure 4) on modifie l'état d'équilibre, donc la forme, de la partie optique de l'implant, donc sa forme et le rayon de courbure R3 de sa face antérieure 1a est modifiée. La puissance optique de la lentille composite 1, 2 est donc modifiée.

Dans le cas de la figure 4 le champ de forces F résulte de l'action du sac capsulaire sur les semelles 5 et 6 lorsque les fibres zonulaires sont relâchées et que, par élasticité naturelle, le sac se contracte radialement. Il s'agit d'un état de l'implant pour une vision de près, le rayon R3 étant plus petit que le rayon R2 qui, lui, correspond sensiblement à la forme de la lentille pour une vision de loin.

Bien entendu la face postérieure 1b de la coque 1 voit son rayon de courbure se modifier sous l'effet d'une modification de l'équilibre des forces. On peut, en jouant par exemple sur des variations d'épaisseurs de la coque 1, favoriser telle ou telle déformation, antérieure ou postérieure, ou même tel type de déformée (sphérique, conique...) . A la figure 7 on a représenté des fentes 7 ménagées dans la paroi antérieure de la coque 1 pour illustrer un moyen d'ajustement de la capacité de cette coque à se déformer.

Aux figures 5 et 6, la variante d'implant représentée illustre une coque 10 qui, vide, possède un volume V plus renflé que celui du noyau 20 qu'elle doit emprisonner. Cette différence de formes engendre, comme précédemment, des champs de forces qui à l'équilibre se traduisent par une forme intermédiaire du noyau emprisonné dans la coque.

Le noyau 2, 20 peut être constitué par un gel non élastique enfermé dans une enveloppe ou une poche élastique qui lui confère d'une part sa forme et d'autre part sa capacité à être élastiquement déformé.

## Revendications

1. Implant intracapsulaire accommodatif comprenant une partie optique centrale et au moins deux bras haptiques (3, 4) dont les extrémités libres (5, 6) sont conformées pour coopérer avec des portions de zone équatoriale du sac capsulaire, la partie optique étant formée par la réunion de deux corps élastiquement déformables l'un en forme d'enveloppe (1) et l'autre en forme de noyau (2), **caractérisé en ce que** l'enveloppe présente une surface extérieure avec une face antérieure convexe (1a) qui possède, lorsque l'enveloppe est vide un premier rayon de courbure (R1) et lorsque l'enveloppe emprisonne le noyau (2), un deuxième rayon de courbure (R2) différent du premier rayon de courbure (R1).

2. Implant selon la revendication 1, **caractérisé en ce que** le volume intérieur (V) de l'enveloppe (1) est identique au volume du noyau (2) mais de forme propre différente de celle de ce noyau, lorsque l'enveloppe et le noyau sont à l'état libre.

3. Implant selon l'une quelconque des revendications 1 et 2, **caractérisé en ce que** le noyau (2) est composé d'un gel enfermé dans une enveloppe élastique qui détermine sa forme propre.

4. Implant selon l'une quelconque des revendications 1 à 3, **caractérisé en ce que** le noyau (2) est formé d'un matériau élastomère de forme déterminée.

5. Implant selon la revendication 4, **caractérisé en ce que** la forme propre du volume intérieur de l'enveloppe (1) à vide et la forme propre à l'état libre du noyau (2) sont telles que lorsque les deux corps sont assemblés, ils sont élastiquement déformés de manière antagoniste pour adopter une forme commune dans laquelle le noyau et l'enveloppe s'opposent mutuellement à leur tendance respective à recouvrer élastiquement leur propre forme.

6. Implant selon l'une quelconque des revendications précédentes, **caractérisé en ce que** la paroi antérieure de l'enveloppe est pourvue d'au moins une fente radiale (7).

## Claims

1. Accommodative intracapsular implant including a central optical part and at least two haptic arms (3, 4) of which the free ends (5, 6) are formed to cooperate with portions of the equatorial zone of the capsular bag, the optical part being formed by the joining-together of two elastically deformable bodies, one shaped as an envelope (1) and the other shaped as a core (2), **characterised in that** the envelope has an external surface with a convex anterior face (1a) which has, when the envelope is empty, a first radius of curvature (R1) and, when the envelope houses the core (2), a second radius of curvature (R2) different from the first radius of curvature (R1).

2. Implant according to claim 1, **characterised in that** the interior volume (V) of the envelope (1) is identical to the volume of the core (2) but has an inherent shape different from that of this core, when the envelope and the core are in the free state.

3. Implant according to any one of claims 1 and 2, **characterised in that** the core (2) is composed of a gel enclosed in a resilient envelope which determines its inherent shape.

4. Implant according to any one of claims 1 to 3, **characterised in that** the core (2) is made of an elastomeric material having a given shape.

5. Implant according to claim 4, **characterised in that** the inherent shape of the interior volume of the envelope (1) when empty and the inherent shape of the core (2) in the free state are such that, when the two bodies are assembled, they are elastically deformed in an antagonistic way to adopt a common shape in which the core and the envelope mutually act against their respective tendency to elastically recover their inherent shape.

6. Implant according to any one of the preceding claims, **characterised in that** the anterior wall of the envelope is provided with at least one radial slit (7).

## Patentansprüche

1. Anpassbares, intrakapsuläres Implantat, welches einen mittigen optischen Teil und mindestens zwei haptische Arme (3,4) umfasst, deren freibewegliche Enden (5,6) derart gestaltet sind, dass sie mit Abschnitten des äquatorialen Bereichs des kapsulären Sacks zusammenwirken, wobei der optische Teil durch die Vereinigung von zwei elastisch verformbaren Körpern gebildet ist, von denen einer als Hülle (1) ausgebildet ist und der andere als Kern (2), **dadurch gekennzeichnet, dass** die Hülle eine Außenfläche mit einer konvexen Vorderseite (1a) umfasst, welche, wenn die Hülle leer ist, einen ersten Bogenradius (R1) aufweist und wenn die Hülle den Kern (2) umfasst, einen zweiten Bogenradius (R2) aufweist, welcher sich von dem ersten Bogenradius (R1) unterscheidet.

2. Implantat nach Anspruch 1, **dadurch gekennzeichnet, dass** das Innenvolumen (V) der Hülle (1) dem Volumen des Kernes (2) entspricht, aber eine eigene, vom Kern unterschiedliche Form aufweist, wenn die Hülle und der Kern freibeweglich sind.

3. Implantat nach einem der Ansprüche 1 und 2, **dadurch gekennzeichnet, dass** der Kern (2) aus einem in einer elastischen Hülle eingeschlossenen Gel besteht, welche seine eigene Form bestimmt.

4. Implantat nach einem der Ansprüche 1 bis 3, **dadurch gekennzeichnet, dass** der Kern (2) aus einem Elastomerstoff mit einer bestimmten Form gebildet ist.

5. Implantat nach Anspruch 4, **dadurch gekennzeichnet, dass** die eigene Form des Innenvolumens der Hülle (1), wenn diese leer ist, und die eigene Form des Kerns (2), wenn dieser frei ist, derart sind, dass wenn die beiden Körper zusammengefügt sind, sie derart elastisch gegensätzlich verformbar sind, dass sie eine gemeinsame Form annehmen können, in welcher der Kern und die Hülle ihren jeweiligen Tendenzen, ihre eigene Form wiedereinzunehmen, gegenseitig entgegenwirken.

6. Implantat nach einem der vorangehenden Ansprüche, **dadurch gekennzeichnet, dass** die Vorderwand der Hülle mit mindestens einem radialen Schlitz (7) versehen ist.
